(19) Europäisches Patentamt
European Patent Office
Office européen des brevets

(11) Veröffentlichungsnummer: 0 336 118 A1

(12) # EUROPÄISCHE PATENTANMELDUNG

(21) Anmeldenummer: 89103748.3

(51) Int. Cl.4: C07D 237/14 , A01N 43/58

(22) Anmeldetag: 03.03.89

(30) Priorität: 10.03.88 DE 3807896

(43) Veröffentlichungstag der Anmeldung: 11.10.89 Patentblatt 89/41

(84) Benannte Vertragsstaaten: DE FR GB IT

(71) Anmelder: BASF Aktiengesellschaft
Carl-Bosch-Strasse 38
D-6700 Ludwigshafen(DE)

(72) Erfinder: Freund, Wolfgang, Dr.
Johann-Gottlieb-Fichte-Strasse 71
D-6730 Neustadt(DE)
Erfinder: Hamprecht, Gerhard, Dr.
Rote-Turm-Strasse 28
D-6940 Weinheim(DE)
Erfinder: Wuerzer, Bruno, Dr.
Ruedigerstrasse 13
D-6701 Otterstadt(DE)
Erfinder: Westphalen, Karl-Otto, Dr.
Mausbergweg 58
D-6720 Speyer(DE)
Erfinder: Meyer, Norbert, Dr.
Dossenheimer Weg 22
D-6802 Ladenburg(DE)

(54) **2-Phenylpyridazin-3-on-Verbindungen, Verfahren zur ihrer Herstellung und ihre Verwendung als Herbizide.**

(57) 2-Phenylpyridazin-3-on-Verbindungen der Formeln

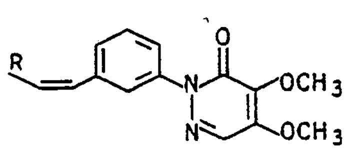
Ia

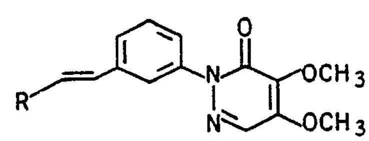
Ib

in der R die folgende Bedeutung hat:
$C_1$-$C_8$-Alkyl;
ggf. substituiertes Benzyl;
ggf. substituiertes $C_3$-$C_8$-Cycloalkyl;
ein 4- bis 6-gliedriger heteroaliphatischer Ring, enthaltend ein Sauerstoff- oder Schwefelatom oder eine N-$CH_3$-Gruppe als Ringglied; ggf. substituiertes Styryl;
ggf. substituierter 5- oder 6-gliedriger aromatischer oder heteroaromatischer Ring, welcher benzokondensiert sein kann, Verfahren zur ihrer Üerstellung und ihre Verwendung.

EP 0 336 118 A1

**2-Phenylpyridazin-3-on-Verbindungen, Verfahren zu ihrer Herstellung und ihre Verwendung als Herbizide**

Die vorliegende Erfindung betrifft 2-Phenylpyridazin-3-on-Verbindungen der Formeln Ia und Ib

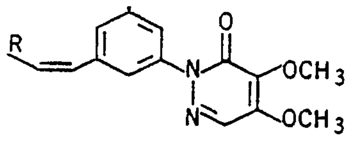

Ia

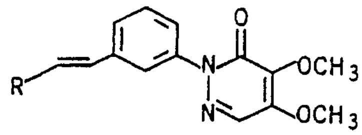

Ib

in der R die folgende Bedeutung hat:
eine $C_1$-$C_8$-Alkylgruppe;
eine Benzylgruppe, wobei der aromatische Ring ein bis drei der folgenden Substituenten tragen kann: Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio und/oder Cyano;
eine $C_3$-$C_8$-Cycloalkylgruppe, welche ein bis drei der folgenden Substituenten tragen kann: Halogen, $C_1$-$C_4$-Alkyl, $C_1$-$C_4$-Halogenalkyl, $C_1$-$C_4$-Alkoxy und/oder $C_1$-$C_4$-Halogenalkoxy;
ein 4- bis 6-gliedriger heteroaliphatischer Ring, enthaltend ein Sauerstoff- oder Schwefelatom oder eine $N$-$CH_3$-Gruppe als Ringglied;
ein Styrylrest, wobei der aromatische Ring ein bis drei der folgenden Substituenten tragen kann: Nitro, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio und/oder Cyano;
ein 5- oder 6-gliedriger aromatischer Ring, welcher ein bis zwei Stickstoff-, Sauerstoff- und/oder Schwefelatome als Ringglieder enthalten kann, wobei dieser Ring zusätzlich benzokondensiert sein kann und ein bis vier der folgenden Substituenten tragen kann:
$C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro, Cyano, Amino, Di-$C_1$-$C_4$-alkylamino, $C_2$-$C_6$-Acylamino, $C_1$-$C_4$-Alkoxycarbonyl, Phenoxy, Phenylthio und/oder eine ortho-ständig an den aromatischen Ring gebundene Brücke
$-X_n-(CH_2)_m-X_n-$,
wobei
X ein Sauerstoff- oder Schwefelatom,
n 1 und gleichzeitig m 1 oder 2 oder
n 0 und gleichzeitig m 3, 4 oder 5
bedeutet,
insbesondere solche Verbindungen Ia und Ib, in denen R die folgende Bedeutung hat:
ein 6-gliedriger aromatischer Ring, wobei dieser Ring zusätzlich benzokondensiert sein kann und ein bis vier der folgenden Substituenten tragen kann:
$C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro, Cyano, Amino, Di-$C_1$-$C_4$-alkylamino, $C_2$-$C_6$-Acylamino, $C_1$-$C_4$-Alkoxycarbonyl, Phenoxy, Phenylthio und/oder eine ortho-ständig an den aromatischen Ring gebundene Brücke
$-X_n-(CH_2)_m-X_n-$,
wobei
X ein Sauerstoff- oder Schwefelatom,
n 1 und gleichzeitig m 1 oder 2 oder
n 0 und gleichzeitig m 3, 4 oder 5
bedeutet.

Des weiteren betrifft die Erfindung Verfahren zur Herstellung der Verbindungen Ia und Ib sowie ihre Verwendung in herbiziden Mitteln zur Bekämpfung unerwünschten Pflanzenwuchses.

Aus der DE-OS 3 617 997 sind Verbindungen der allgemeinen Struktur

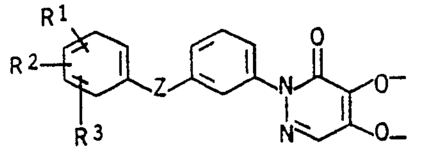

(A)

bekannt, bei der Z die Verknüpfung durch eine $C_1$-$C_5$-Alkylenkette bedeutet.

Aufgabe der vorliegenden Erfindung war die Bereitstellung von Verbindungen, die in ihren biologischen Eigenschaften den bekannten Herbiziden bezüglich der Wirkung gegen unerwünschte Pflanzen überlegen sind, ohne die Kulturpflanzen zu schädigen.

Es wurde nun gefunden, daß die eingangs definierten neuen Verbindungen der Formeln Ia und Ib eine größere Wirksamkeit gegen Schadpflanzen bzw. eine verbesserte Selektivität gegenüber wichtigen Nutzpflanzen besitzen.

Außerdem wurden Verfahren zur Herstellung der Verbindungen Ia und Ib gefunden.

Man erhält die erfindungsgemäßen Verbindungen der Formeln Ia und Ib nach folgenden Verfahren:

A) Durch Wittig-Reaktion eines Phosphoniumsalzes der Formel II in an sich bekannter Weise und einem Aldehyd der Formel III, in der R die in Formel I angegebene Bedeutung besitzt, wobei Verbindungen der Formel I erhalten werden (vgl. Houben-Weyl, Bd. 5/1b, S. 383).

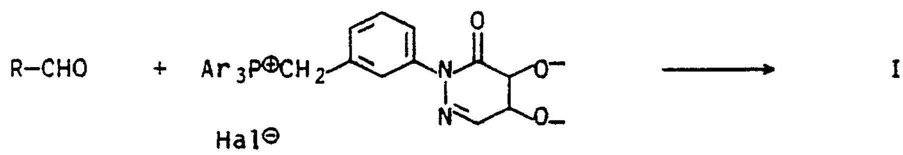

III II

Das Verfahren wird zweckmäßigerweise so durchgeführt, daß man das Ausgangsmaterial der Formel II in einem organischen Lösungsmittel in Gegenwart einer Base mit einem Aldehyd der Formel III im Bereich von 0,5 bis 24 Stunden bei einer Temperatur zwischen 0 °C und der Siedetemperatur des gewählten Lösungsmittels umsetzt. Die Ausgangsmaterialien der Formeln II und III werden zweckmäßig in etwa stöchiometrischer Menge verwendet. Als Lösungsmittel sind besonders Alkohole wie z. B. Methanol oder Ethanol oder Amide wie z. B. Dimethylformamid bevorzugt, als Basen können z. B. Alkalialkoholate eingesetzt werden. Wegen der leichten Zugänglichkeit sind Arylreste in den Verbindungen der Formel II und besonders (ggf. substituierte Phenylreste und als Halogenanionen besonders Chlorid und Bromid bevorzugt. Die Umsetzung ist im allgemeinen nach 4-8 Stunden beendet und es kann wie üblich aufgearbeitet werden: Fällt das Endprodukt fest an, so isoliert man es beispielsweise durch Absaugen des Niederschlags. Ist das Endprodukt dagegen im Lösungsmittel gelöst, wird dieses unter vermindertem Druck abdestilliert. Der Rückstand wird zwischen Wasser und einem organischen Lösungsmittel verteilt und das Zielprodukt durch Einengen erhalten. Zur Reinigung kann beispielsweise umkristallisiert oder chromatographiert werden.

B) Durch Horner-Emmons-Reaktion eines Phosphonats der Formel IV mit einem Aldehyd der Formel III werden ebenfalls Verbindungen der Formel I erhalten (vgl. Houben-Weyl, Bd. 12/1, S. 523, Bd. 5/1b, S. 395).

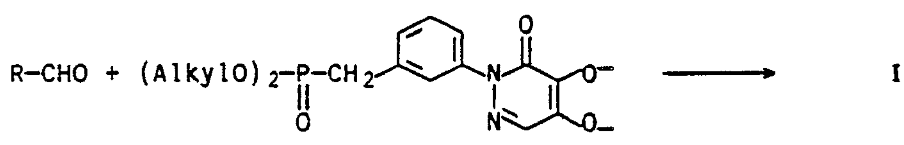

III IV

Das Verfahren wird zweckmäßigerweise so durchgeführt, daß man das Phosphonat, z.B. Methyl- oder Ethylverbindung IV in einem organischen Lösungsmittel in Gegenwart einer Base mit dem Aldehyd III bei einer Temperatur zwischen -20 °C und der Siedetemperatur des verwendeten Lösungsmittels umsetzt. Die Ausgangsmaterialien werden in etwa stöchiometrischer Menge verwendet. Als Lösungsmittel können

übliche organische Lösungsmittel: Ether wie Diethylether oder Tetrahydrofuran oder Amide wie z. B. Dimethylformamid oder aromatische Kohlenwasserstoffe wie z. B. Benzol oder Toluol eingesetzt werden; als Basen werden Alkalimetallhydride bevorzugt. Die Umsetzung ist im allgemeinen nach 4-8, höchstens 24 Stunden beendet. Man arbeitet wie üblich auf: Fällt das Endprodukt fest an, so isoliert man es beispielsweise durch Absaugen des Niederschlags. Ist das Endprodukt dagegen im Lösungsmittel gelöst, wird dieses zwischen Wasser und einem organischen Lösungsmittel verteilt und das Reaktionsprodukt der Formel I durch Einengen des organischen Lösungsmittels erhalten. Zur Reinigung kann das Produkt der Formel I beispielsweise umkristallisiert oder chromatographiert werden.

Phosphoniumverbindung II und Phosphonat IV können wie üblich aus einem entsprechenden Halogenid und einem Triarylphosphin bzw. einem Phosphorigsäuretrialkylester hergestellt werden (vgl. Houben-Weyl, Bd. 12/1, S. 79 bzw. S. 433):
Das Halogenid VI erhält man seinerseits aus der bekannten Methylverbindung (V; vgl. FR-A-2 099 642) durch Halogenierung (vgl. Houben-Weyl Bd. 5/4, S. 331; Bd. 5/3, S. 503, 800; Bd. 4/5a, S. 134).

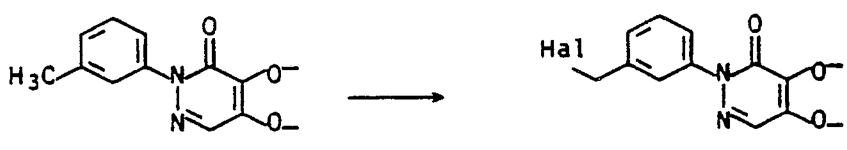

V VI

Als Halogene in den Verbindungen der Formel VI werden Chlor und Brom bevorzugt. Die Halogenierung kann sowohl mit elementarem Halogen, bevorzugt unter gleichzeitiger Belichtung, oder durch Halogenüberträger in Gegenwart von Radikalbildnern wie Dibenzoylperoxid oder 2,2′-Azoisobuttersäuredinitril oder Belichtung ausgeführt werden. Als Halogenüberträger kommen insbesondere N-Halogenverbindungen wie beispielsweise N-Bromsuccinimid oder N-Chlorsuccinimid in Frage.

Die erfindungsgemäßen Verbindungen I stellen in der Regel kristalline oder zähflüssige Verbindungen dar. Sie entstehen nach den angegebenen Verfahren in der Regel als cis/trans-Gemische Ia bzw. Ib, wobei im allgemeinen nach Verfahren B) überwiegend die trans-Verbindungen Ib erhalten werden können. Die Gemische Ia, Ib können im allgemeinen durch Umkristallisation oder Chromatographie in die reinen Verbindungen Ia bzw. Ib getrennt werden.

Im Hinblick auf eine bestimmungsgemäße Verwendung der 2-Phenylpyridazin-3-on-Verbindungen Ia und Ib kommen als Substituent R folgende Reste in Betracht:
eine Alkylgruppe wie Methyl, Ethyl, Propyl, 1-Methylethyl, Butyl, 1-Methylpropyl, 2-Methylpropyl, 1,1-Dimethylpropyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 1-Methylhexyl, 2-Methylhexyl, 3-Methylhexyl, 4-Methylhexyl, 5-Methylhexyl, 1-Ethylpentyl, 2-Ethylpentyl, 1-Propylbutyl und Octyl, insbesondere 1,1-Dimethylethyl, 2,2-Dimethylpropyl und 1,2-Dimethylbutyl;
heteroaliphatische Ringe wie Oxethanyl, Thiethanyl, N-Methyl-azetidinyl, Oxolanyl, Thiolanyl, N-Methylazolidinyl, Dioxolanyl, Thioxolanyl, Dithiolanyl, N-Methyloxazolidin, N-Methylthiazolidin, Tetrahydropyranyl, Tetrahydrothiopyranyl, N-Methylpiperidyl, Tetrahydro-1,3-dioxanyl, Tetrahydro-1,4-dioxanyl, Tetrahydro-1,3-thioxanyl, Tetrahydro-1,4-thioxanyl, Tetrahydro-1,3-dithianyl, N-Methylmorpholinyl, N-Methyl-hexahydro-1,3-oxazin, N-Methyl-hexahydro-1,3-thiazin und N-Methyl-hexahydro-1,4-thiazin;
ein Benzylrest, ein Phenylrest, ein Naphthylrest, ein Styrylrest oder eine der folgenden heteroaromatischen Gruppen: 2-, 3- oder 4-Pyridyl, 2-,4- oder 5-Pyrimidyl, 2- oder 3-Pyrazinyl, 2-, 3- oder 4-Chinolyl, 2- oder 3-Thienyl, 2- oder 3-Furyl, 2- oder 3-Pyrrolyl, 2-, 4- oder 5-Thiazolyl, 2-, 4- oder 5-Imidazolyl, 2-, 4- oder 5-Oxazolyl, 3-, 4- oder 5-Isothiazolyl, 3-, 4- oder 5-Pyrazolyl, 3-, 4- oder 5-Isoxazolyl, 2-Thiadiazolyl, 2-Oxadiazolyl, 2-Benzthiazolyl-, 2-Benzoxazolyl oder 2-Benzimidazolyl.

Diese aromatischen Ringe können jeweils unsubstituiert oder ein- bis dreifach substituiert sein. Als Substituenten kommen dabei folgende Gruppen in Betracht:

Cyanogruppen,
Halogenatome wie Fluor, Chlor, Brom und Iod, insbesondere Fluor, Chlor und Brom;
$C_1$-$C_4$-Alkylgruppen wie obengenannt, insbesondere Methyl, Ethyl und 1-Methylethyl;

Halogenalkylgruppen wie Fluormethyl, Difluormethyl, Trifluormethyl, Chlordifluormethyl, Dichlorfluormethyl, Trichlormethyl, 1-Fluorethyl, 2-Fluorethyl, 2,2-Difluorethyl, 2,2,2-Trifluorethyl, 2-Chlor-2,2-difluorethyl, 2,2-Dichlor-2-fluorethyl, 2,2,2-Trichlorethyl und Pentafluorethyl, insbesondere Difluormethyl, Trifluormethyl, 2,2,2-Trifluorethyl und Pentafluorethyl;
Alkoxygruppen wie Methoxy, Ethoxy, Propoxy, 1-Methylethoxy, Butoxy, 1-Methylpropoxy, 2-Methylpropoxy und 1,1-Dimethylethoxy, insbesondere Methoxy, Ethoxy, 1-Methylethoxy und 1,1-Dimethylethoxy;
Halogenalkoxygruppen wie Difluormethoxy, Trifluormethoxy, Chlordifluormethoxy, Dichlorfluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy und Pentafluorethoxy, insbesondere Trifluormethoxy;
Alkylthiogruppen wie Methylthio, Ethylthio, Propylthio, 1-Methylethylthio, Butylthio, 1-Methylpropylthio, 2-Methylpropylthio und 1,1-Dimethylethylthio, insbesondere Methylthio und Ethylthio;
Halogenalkylthiogruppen wie Difluormethylthio, Trifluormethylthio, Chlordifluormethylthio, Dichlorfluormethylthio, 1-Fluorethylthio, 2-Fluorethylthio, 2,2-Difluorethylthio, 1,1,2,2-Tetrafluorethylthio, 2,2,2-Trifluorethylthio, 2-Chlor-1,1,2-trifluorethylthio und Pentafluorethylthio, insbesondere Trifluormethylthio.

Der Styrylrest kann neben den obengenannten Gruppen auch durch Nitrogruppen substituiert sein, während die 5- oder 6-gliedrigen aromatischen und heteroaromatischen Reste überdies hinaus noch folgende Substituenten tragen können:
Phenoxygruppen, Phenylthiogruppen, Aminogruppen, welche zweifach durch die obengenannten $C_1$-$C_4$-Alkylgruppen oder einfach oder zweifach durch Acylgruppen wie Acetyl, Propionyl, iso-Propionyl, Butyryl, 2-Methylpropionyl, Pentanoyl, 2-Methylbutyryl, 3-Methylbutyryl, 2,2-Dimethylpropionyl, Hexanoyl, 2-Methylpentanoyl, 3-Methylpentanoyl, 4-Methyl pentanoyl, 2,2-Dimethylbutyryl, 2,3-Dimethylbutyryl, 3,3-Dimethylbutyryl und 2-Ethylbutyryl, insbesondere Acetyl und Propionyl, substituiert sein können und
Alkoxycarbonylgruppen wie Methoxycarbonyl, Ethoxycarbonyl, Propyloxycarbonyl, 1-Methylethoxycarbonyl, Butyloxycarbonyl, 1-Methylpropylcarbonyl, 2-Methylpropylcarbonyl und 1,1-Dimethylethoxycarbonyl, insbesondere Methoxycarbonyl und Ethoxycarbonyl.

Daneben können benachbarte Ringpositionen dieser aromatischen Ringe durch eine Brücke $-X_n-(CH_2)_m-X_n-$verbunden sein, wobei ein X Sauerstoff- oder Schwefelatom bedeutet und n den Wert 1 besitzt, wenn m 1 oder 2 bedeutet bzw. n den Wert 0 besitzt, wenn m 3, 4 oder 5 bedeutet. Beispiele für derartige Brücken sind im folgenden aufgeführt:
$-O-CH_2-O$, $-O-CH_2-O-$, $-S-CH_2-S-$, $-S-CH_2CH_2-S-$,
$-CH_2CH_2CH_2-$, $-CH_2CH_2CH_2CH_2-$ und $-CH_2CH_2CH_2CH_2CH_2-$
Die 2-Phenylpyridazin-3-on-Verbindungen Ia und/oder Ib bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Verbindungen Ia und Ib eignen sich allgemein zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol,

Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff.

Die Wirkstoffe können beispielsweise wie folgt formuliert werden:

I. Man vermischt 90 Gewichtsteile der Verbindung Nr. 19 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 47a. werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 75 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 104 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 137 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 155 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 166 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 164 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5,0, vorzugsweise 0,01 bis 0,5 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |

| Botanischer Name | Deutscher Name |
|---|---|
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Verbindungen der Formel Ia und Ib mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Phenyloxy- bzw. Heteroaryloxy-phenylpropionsäuren sowie deren Salze, Ester und Amide und andere in Betracht.

Außerdem kann es von Nutzen sein, die neuen Verbindungen der Formel Ia und Ib allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Synthesebeispiele:

Die in den nachstehenden Synthesebeispielen wiedergegebenen Vorschriften wurden unter entsprechender Abwandlung der Ausgangsverbindungen zur Gewinnung weiterer Verbindungen der Formel Ia und Ib benutzt; die erhaltenen Verbindungen sind in den nachfolgenden Tabellen mit physikalischen Angaben aufgeführt; Verbindungen ohne diese Angaben lassen sich aus den entsprechenden Stoffen in analoger Weise aufbauen. Sie lassen aufgrund ihrer nahen strukturellen Beziehungen zu den hergestellten und untersuchten Verbindungen eine gleichartige Wirkung erwarten.

Beispiel 1

Herstellweg über die Wittig-Verbindung

A) Zu einer Lösung aus 105 g 1-(3′-Methylphenyl)-4,5-dimethoxypyridazinon-(6) und 51,6 g Natriumacetat in 1,5 l Dichlormethan und 500 ml Wasser werden unter Belichtung mit einer 300 W-Glühlampe und kräftigem Rühren eine Lösung von 80,0 g Brom in 400 ml Dichlormethan zugetropft. Sodann wird Dichlormethan abgetrennt, mit Natriumcarbonatlösung gewaschen und eingeengt. Man erhält 124 g 1-(3′-Brommethylphenyl)-4,5-dimethoxypyridazinon-(6) vom Fp. 67-69 °C und einer Reinheit von 90 %.

B) Eine Lösung aus 120 g 1-(3′-Brommethylphenyl)-4,5-dimethoxypyridazinon-(6) (85 proz. Reinheit) und 97,0 g Triphenylphosphin in 500 ml Aceton werden 2,5 Stunden zum Sieden erhitzt. Durch Absaugen erhält man 167 g 3-(4′,5′-Dimethoxypyridazinon-(6′))-phenylmethyltriphenylphosphoniumbromid vom Fp. 217 °C (Zers.).

C) Eine Suspension aus 15,0 g 1-(3′-Methylphenyl)-4,5-dimethoxypyridazinon-(6), 12,2 g N-Chlorsuccinimid und 2,3 g Dibenzoylperoxid in 150 ml Chlorbenzol wird 3 Stunden bei Siedetemperatur gerührt, eingeengt, in Toluol aufgenommen und an Kieselgel chromatographiert. Man erhält 5,6 g braune Kristalle, die nach $^1$H-NMR aus 50 % Ausgangsmaterial, 35 % 1-(3′-Chlormethyl-phenyl)-4,5-dimethoxypyridazinon-(6) und 15 % 1-(3′-Dichlormethylphenyl)-4,5-dimethoxypyridazinon-(6) bestehen und getrennt und gereinigt werden.

D) 9,56 g 1-(3′-Chlormethylphenyl)-4,5-dimethoxypyridazinon-(6) (88 proz. Reinheit) und 7,85 g Triphenylphosphin werden in 100 ml Toluol 8 Stunden auf 80 °C und 5 Stunden auf Siedetemperatur erhitzt, eingeengt und der Rückstand mit Aceton verrieben. Man erhält 9 g 3-(4′,5′-Dimethoxypyridazinon-(6′))-phenylmethyl-triphenylphosphoniumchlorid vom Fp. 198-201 °C.

E) Zu einer Suspension von 23,5 g 3-(4′,5′-Dimethoxypyridazinon-(6′))-phenylmethyl-triphenylphosphoniumbromid in 100 ml Methanol werden 7,20 g 30 proz. Natriummethylat in Methanol getropft und 20 min bei Raumtemperatur nachgerührt. Anschließend tropft man 4,20 g Benzaldehyd zu, rührt 6 Stunden bei Raumtemperatur, verdünnt mit Wasser und schüttelt mit Essigsäureethylester aus. Das nach dem Einengen verbleibende Öl wird an Kieselgel (Cyclohexan/Essigsäureethylester) chromatographiert. Man erhält 10,6 g cis- und trans-3-(4′,5′-Dimethoxypyridazinon-(6′))-stilben als farbloses Öl.

Die cis/trans-Isomeren können durch nochmalige Chromatographie getrennt werden (Cyclohexan/Essigsäureethylester). Man erhält als leichter eluierbare Substanz cis-3-(4′,5′-Dimetho

xypyridazinon-(6'))-stilben vom Fp. 48-51 °C (Verbindung Nr. 1).

Als zweite Fraktion erhält man trans-3-(4',5'-Dimethoxypyridazinon-(6'))-stilben vom Fp. 87-91 °C (Verbindung Nr. 2).

Herstellweg über das Phosphonat

A) Eine Suspension aus 152 g 1-(3'-Methylphenyl)-4,5-dimethoxypyridazinon-(6), 165 g N-Bromsuccinimid und 10 g 2,2'-Azoisobuttersäuredinitril in 1 l 1,1,1-Trichlorethan wird 5 Stunden zum Rückfluß erhitzt. Nach dem Abkühlen wird abgesaugt und das Filtrat eingeengt. Man erhält 252 g braune Kristalle vom Fp. 53-58 °C, die nach $^1$H-NMR zu 73 % aus 1-(3'-Brommethylphenyl)-4,5-dimethoxypyridazinon-(6) bestehen.

B) 40 g 1-(3'Brommethylphenyl)-4,5-dimethoxypyridazinon-(6) (nach $^1$H-NMR 50 proz. Reinheit) und 66 g Phosphorigsäuretriethylester werden auf 120 °C erhitzt. Nach 1 Stunde wird der Überschuß an Phosphorigsäuretriethylester im Vakuum entfernt und der Rückstand an Kieselgel chromatographiert (Essigester/Ethanol = 90/10). Man erhält 20,2 g 3-(4',5'-Dimethoxypyridazinon-(6'))-phenylmethylphosphonsäurediethylester als gelbes Öl, das nach Anreiben mit Ether/Pentan kristallisiert. Der Fp. der farblosen Kristalle beträgt 71-72 °C.

C) Zu 1,15 g Natriumhydrid werden 10,6 g Phosphonat aus Beispiel B in 50 ml absolutem Tetrahydrofuran zugetropft. Nach 30 Minuten tropft man bei Raumtemperatur 3,18 g Benzaldehyd gelöst in 50 ml absolutem Tetrahydrofuran zu. Man rührt über Nacht nach, gießt auf Wasser und schüttelt mit Diethylether aus. Einengen liefert 8,8 g trans-3-(4',5'-Dimethoxypyridazinon-(6'))-stilben vom Fp. 89-90 °C.

Beispiel 2

Zu 5,30 g Ethanolamin werden bei 80 °C 3,40 g der in der folgenden Tabelle als Nr. 157 angegebenen Verbindung zugegeben, 10 Minuten bei 80 °C nachgerührt und zwischen Wasser und Essigsäureethylester verteilt. Der nach dem Einengen des Essigsäureethylesters verbleibende Rückstand wird mit Wasser verrührt und anschließend an Kieselgel (Toluol/Aceton) chromatographiert. Man erhält 1-Amino-4(3'(4'',5''-dimethoxypyridazinon-(6''))-stilben als farbloses Öl (Verbindung Nr. 3), cis/trans-Gemisch:H-NMR:cis:7,80 (S; 1 H); trans:7,85 (S; 1 H).

Tabelle 1

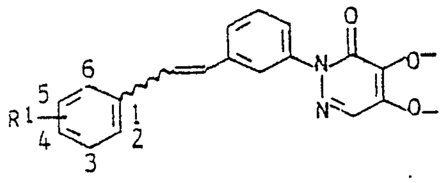

| Nr. | $R^1$ | Fp (°C) | cis/trans-Verhältnis | H-NMR (ppm) |
|---|---|---|---|---|
| 4 | 2-$CH_3$ | | | |
| 5 | 3-$CH_3$ | | | |
| 6 | 4-$CH_3$ | | | |
| 7 | 2-$C_2H_5$ | | | |
| 8 | 3-$C_2H_5$ | | | |
| 9 | 4-$C_2H_5$ | | | |
| 10 | 3-n-$C_3H_7$ | | | |
| 11 | 4-n-$C_3H_7$ | | | |
| 12 | 3-i-$C_3H_7$ | | | |
| 13 | 4-i-$C_3H_7$ | | | |
| 14 | 3-n-$C_4H_9$ | | | |
| 15 | 4-n-$C_4H_9$ | | | |
| 16 | 3-sec-$C_4H_9$ | | | |
| 17 | 4-sec-$C_4H_9$ | | | |
| 18 | 3-t-$C_4H_9$ | | | 7,79 (s; 1 H) |
| 19 | 4-t-$C_4H_9$ | Oel | cis | |
| 20 | 4-t-$C_4H_9$ | 132-140 | trans | |
| 21 | 3-n-$C_5H_{11}$ | | | |
| 22 | 4-n-$C_5H_{11}$ | | | |
| 23 | 3-n-$C_6H_{13}$ | | | |
| 24 | 4-n-$C_6H_{13}$ | | | |
| 25 | 3-Phenyl | | | |
| 26 | 4-Phenyl | | | |
| 27 | 2,3-$(CH_3)_2$ | | | |
| 28 | 2,4-$(CH_3)_2$ | | | |
| 29 | 2,5-$(CH_3)_2$ | | | |

Tabelle 1 (Fortsetzung):

| Nr. | $R^1$ | Fp (°C) | cis/trans-Verhältnis | H-NMR (ppm) |
|---|---|---|---|---|
| 30 | 2,6-$(CH_3)_2$ | | | |
| 31 | 3,4-$(CH_3)_2$ | | | |
| 32 | 3,5-$(CH_3)_2$ | | | |
| 33 | 2,3,4-$(CH_3)_3$ | | | |
| 34 | 2,3,5-$(CH_3)_3$ | | | |
| 35 | 2,4,5-$(CH_3)_3$ | | | |
| 36 | 2,4,6-$(CH_3)_3$ | | | |
| 37 | 3,4,5-$(CH_3)_3$ | | | |
| 38 | 2-$CF_3$ | Oel | cis/trans = 2/1 | cis: 7,77 (s;1H); trans: 7,84 (s;1H) |
| 39 | 3-$CF_3$ | Oel | cis/trans = 1/1 | cis: 7,79 (s;1H); trans: 7,86 (s;1H) |
| 40 | 4-$CF_3$ | 61 | cis | |
| 41 | 4-$CF_3$ | 88-94 | trans | |
| 42 | 2-F | 75-80 | cis/trans = 2/1 | |
| 43 | 3-F | Oel | cis | 7,79 (s; 1 H) |
| 44 | 3-F | 105-110 | cis/trans = 1/3 | |
| 45 | 4-F | Oel | cis | 7,81 (s; 1 H) |
| 46 | 4-F | 107-112 | trans | |
| 47 | 2-Cl | Oel | cis/trans = 1/1 | cis: 7,77 (s; 1H) trans: 7,84 (s; 1H) |
| 47a | 2-Cl | Oel | cis | 7,77 (s; 1 H) |
| 48 | 3-Cl | Oel | cis | 7,78 (s; 1 H) |
| 49 | 3-Cl | 119-126 | trans | |
| 50 | 4-Cl | 50-53 | cis | |
| 51 | 4-Cl | 114-118 | trans | |
| 52 | 2-Br | | | |
| 53 | 3-Br | | | |
| 54 | 4-Br | 119-123 | cis/trans = 1/1 | |
| 55 | 2,3-$F_2$ | | | |
| 56 | 2,4-$F_2$ | | | |
| 57 | 2,5-$F_2$ | | | |
| 58 | 2,6-$F_2$ | | | |
| 59 | 2,3-$Cl_2$ | | | |
| 60 | 2,4-$Cl_2$ | Oel | cis/trans = 1/2 cis: 7,78 (s; 1H) trans: 7,82 (s; 1H) | |
| 60a | 2,4-$Cl_2$ | 108-110 | cis | |
| 61 | 2,5-$Cl_2$ | | | |

Tabelle 1 (Fortsetzung):

| Nr. | $R^1$ | Fp ($^oC$) | cis/trans-Verhältnis | H-NMR (ppm) |
|---|---|---|---|---|
| 62 | 2,6-$Cl_2$ | | | |
| 63 | 3,4-$Cl_2$ | | | |
| 64 | 3,5-$Cl_2$ | | | |
| 65 | 2,3,4-$Cl_3$ | | | |
| 66 | 2,3,5-$Cl_3$ | | | |
| 67 | 2,4,6-$Cl_3$ | | | |
| 68 | 3,4,5-$Cl_3$ | | | |
| 69 | 2-CN | | | |
| 70 | 3-CN | | | |
| 71 | 4-CN | 165-168 | trans | |
| 72 | 4-CN | 56-60 | cis | |
| 73 | 2-$OCH_3$ | | | |
| 74 | 3-$OCH_3$ | | | |
| 75 | 4-$OCH_3$ | Oel | cis/trans = 2/1 | cis: 7,80 (s;1H); trans: 7,86 (s;1H) |
| 76 | 2-$OC_2H_5$ | | | |
| 77 | 3-$OC_2H_5$ | | | |
| 78 | 4-$OC_2H_5$ | | | |
| 79 | 3-O-n-$C_3H_7$ | | | |
| 80 | 4-O-n-$C_3H_7$ | | | |
| 81 | 3-O-i-$C_3H_7$ | | | |
| 82 | 4-O-i-$C_3H_7$ | | | |
| 83 | 3-O-n-$C_4H_9$ | | | |
| 84 | 4-O-n-$C_4H_9$ | | | |
| 85 | 3-O-t-$C_4H_9$ | | | |
| 86 | 4-O-t-$C_4H_9$ | | | |
| 87 | 4-O-n-$C_5H_{11}$ | | | |
| 88 | 4-O-n-$C_6H_{13}$ | | | |
| 89 | 3-$OC_6H_5$ | | | |
| 90 | 4-$OC_6H_5$ | | | |
| 91 | 2,3-$(OCH_3)_2$ | | | |
| 92 | 2,4-$(OCH_3)_2$ | | | |
| 93 | 2,5-$(OCH_3)_2$ | | | |
| 94 | 2,6-$(OCH_3)_2$ | | | |
| 95 | 3,4-$(OCH_3)_2$ | | | |
| 96 | 3,5-$(OCH_3)_2$ | | | |
| 97 | 3,4,5-$(OCH_3)_3$ | | | |
| 98 | 2-$OCF_3$ | | | |
| 99 | 3-$OCF_3$ | | | |

Tabelle 1 (Fortsetzung):

| Nr. | $R^1$ | Fp (°C) | cis/trans-Verhältnis | H-NMR (ppm) |
|---|---|---|---|---|
| 100 | 4-$OCF_3$ | | | |
| 101 | 2-$OCF_2CHF_2$ | | | |
| 102 | 3-$OCF_2CHF_2$ | | | |
| 103 | 4-$OCF_2CHF_2$ | Oel | cis | 7,78 (s; 1 H) |
| 104 | 4-$OCF_2CHF_2$ | 91-101 | trans | |
| 105 | 2-O-Benzyl | | | |
| 106 | 3-O-Benzyl | | | |
| 107 | 4-O-Benzyl | | | |
| 108 | 3,4-$OCH_2O$ | | | |
| 109 | 3,4-$OCH_2CH_2O$ | | | |
| 110 | 3,4-$SCH_2O$ | | | |
| 111 | 3,4-$SCH_2CH_2O$ | | | |
| 112 | 3,4-$SCH_2S$ | | | |
| 113 | 3,4-$SCH_2CH_2S$ | | | |
| 114 | 2-$SCH_3$ | | | |
| 115 | 3-$SCH_3$ | | | |
| 116 | 4-$SCH_3$ | | | |
| 117 | 2-$SC_2H_5$ | | | |
| 118 | 3-$SC_2H_5$ | | | |
| 119 | 4-$SC_2H_5$ | | | |
| 120 | 3-S-n$C_3H_7$ | | | |
| 121 | 4-S-n$C_3H_7$ | | | |
| 122 | 3-S-i-$C_3H_7$ | | | |
| 123 | 4-S-i-$C_3H_7$ | | | |
| 124 | 3-S-n-$C_4H_9$ | | | |
| 125 | 4-S-n-$C_4H_9$ | | | |
| 126 | 3-S-t-$C_4H_9$ | | | |
| 127 | 4-S-t-$C_4H_9$ | | | |
| 128 | 4-S-n-$C_5H_{11}$ | | | |
| 129 | 4-S-n-$C_6H_{13}$ | | | |
| 130 | 2,4-$(SCH_3)_2$ | | | |
| 131 | 2,5-$(SCH_3)_2$ | | | |
| 132 | 2-$SCF_3$ | | | |
| 133 | 3-$SCF_3$ | | | |
| 134 | 4-$SCF_3$ | | | |
| 135 | 2-$NO_2$ | | | |
| 136 | 3-$NO_2$ | | | |
| 137 | 4-$NO_2$ | 96-105 | cis | |
| 138 | 4-$NO_2$ | 136 | trans | |

Tabelle 1 (Fortsetzung):

| Nr. | $R^1$ | Fp (°C) | cis/trans-Verhältnis | H-NMR (ppm) |
|---|---|---|---|---|
| 139 | 2,3-$(NO_2)_2$ | | | |
| 140 | 2,4-$(NO_2)_2$ | 102-110 | cis | |
| 141 | 2,5-$(NO_2)_2$ | | | |
| 142 | 2,6-$(NO_2)_2$ | | | |
| 143 | 3,4-$(NO_2)_2$ | | | |
| 144 | 3,5-$(NO_2)_2$ | | | |
| 145 | 3-$NH_2$ | | | |
| 146 | 2-$N(CH_3)_2$ | | | |
| 147 | 3-$N(CH_3)_2$ | | | |
| 148 | 4-$N(CH_3)_2$ | | | |
| 149 | 2-$N(C_2H_5)_2$ | | | |
| 150 | 3-$N(C_2H_5)_2$ | | | |
| 151 | 4-$N(C_2H_5)_2$ | Oel | cis/trans = 1/1 | cis:7,78 (s; 1 H); trans: 7,83 (s; 1 H) |
| 152 | 2-$NHCOCH_3$ | | | |
| 153 | 3-$NHCOCH_3$ | | | |
| 154 | 4-$NHCOCH_3$ | | | |
| 155 | 3-Phthalimido | 126-131 | cis | |
| 156 | 3-Phthalimido | 146-151 | cis/trans = 2/8 | |
| 157 | 4-Phthalimido | 104-111 | cis/trans = 2/1 | |
| 158 | 2-$NHCOC_3H_7$ | | | |
| 159 | 3-$NHCOC_3H_7$ | | | |
| 160 | 4-$NHCOC_3H_7$ | | | |
| 161 | 2-COOMe | | | |
| 162 | 3-COOMe | | | |
| 163 | 4-COOMe | | | |

Tabelle 1a

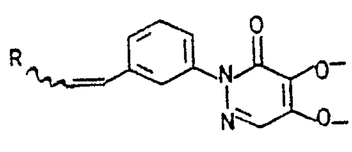

| Nr. | R | Fp (°C) | cis/trans-Verhältnis | H-NMR (ppm) |
|---|---|---|---|---|
| 164 | 1-Naphthyl | 78-72 | cis/trans = 1/1 | |
| 165 | 2-Naphthyl | | | |

Tabelle 2a:

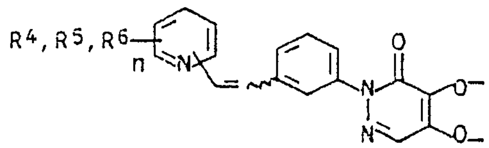

| Nr. | $R^4$, $R^5$, $R^6$ | Verknüpfung | Fp (°C) | cis/trans-Verhältnis | H-NMR (ppm) |
|---|---|---|---|---|---|
| 166 | H | 2 | Oel | cis | 7,81 (s; 1 H) |
| 167 | H | 2 | Oel | trans | 7,88 (s; 1 H) |
| 168 | H | 3 | Oel | cis/trans=1/1 | cis:7,80(s;1H); trans:7,87(s;1H) |
| 169 | H | 4 | Oel | cis/trans=2/1 | cis:7,80 (s;1H) trans:7,89(s;1H) |
| 170 | 3-$CH_3$ | 2 | | | |
| 171 | 4-$CH_3$ | 2 | | | |
| 172 | 5-$CH_3$ | 2 | | | |
| 173 | 6-$CH_3$ | 2 | | | |
| 174 | 2-$CH_3$ | 3 | | | |
| 175 | 4-$CH_3$ | 3 | | | |
| 176 | 5-$CH_3$ | 3 | | | |
| 177 | 6-$CH_3$ | 3 | | | |
| 178 | 2-$CH_3$ | 4 | | | |
| 179 | 3-$CH_3$ | 4 | | | |
| 180 | 3-F | 2 | | | |
| 181 | 4-F | 2 | | | |
| 182 | 5-F | 2 | | | |
| 183 | 6-F | 2 | | | |
| 184 | 3-Cl | 2 | | | |
| 185 | 4-Cl | 2 | | | |
| 186 | 5-Cl | 2 | | | |
| 187 | 6-Cl | 2 | | | |
| 188 | 2-Cl | 3 | | | |
| 189 | 4-Cl | 3 | | | |
| 190 | 5-Cl | 3 | | | |
| 191 | 6-Cl | 3 | | | |
| 192 | 2-Cl | 4 | | | |
| 193 | 3-Cl | 4 | | | |
| 194 | 3-$CF_3$ | 2 | | | |
| 195 | 4-$CF_3$ | 2 | | | |

Tabelle 2a (Fortsetzung)

| Nr. | $R^4$, $R^5$, $R^6$ | Verknüpfung | Fp (°C) | cis/trans-Verhältnis | H-NMR (ppm) |
|---|---|---|---|---|---|
| 196 | 5-$CF_3$ | 2 | | | |
| 197 | 6-$CF_3$ | 2 | | | |
| 198 | 2-$CF_3$ | 3 | | | |
| 199 | 4-$CF_3$ | 3 | | | |
| 200 | 5-$CF_3$ | 3 | | | |
| 201 | 6-$CF_3$ | 3 | | | |
| 202 | 2-$CF_3$ | 4 | | | |
| 203 | 3-$CF_3$ | 4 | | | |
| 204 | 3-$OCH_3$ | 2 | | | |
| 205 | 4-$OCH_3$ | 2 | | | |
| 206 | 5-$OCH_3$ | 2 | | | |
| 207 | 6-$OCH_3$ | 2 | | | |
| 208 | 2-$OCH_3$ | 3 | | | |
| 209 | 4-$OCH_3$ | 3 | | | |
| 210 | 5-$OCH_3$ | 3 | | | |
| 211 | 6-$OCH_3$ | 3 | | | |
| 212 | 2-$OCH_3$ | 4 | | | |
| 213 | 3-$OCH_3$ | 4 | | | |
| 214 | 3-$OCF_3$ | 2 | | | |
| 215 | 4-$OCF_3$ | 2 | | | |
| 216 | 5-$OCF_3$ | 2 | | | |
| 217 | 6-$OCF_3$ | 2 | | | |
| 218 | 3-$SCH_3$ | 2 | | | |
| 219 | 4-$SCH_3$ | 2 | | | |
| 220 | 5-$SCH_3$ | 2 | | | |
| 221 | 6-$SCH_3$ | 2 | | | |
| 222 | 3-$SCF_3$ | 2 | | | |
| 223 | 4-$SCF_3$ | 2 | | | |
| 224 | 5-$SCF_3$ | 2 | | | |
| 225 | 6-$SCF_3$ | 2 | | | |
| 226 | 3-$NO_2$ | 2 | | | |
| 227 | 4-$NO_2$ | 2 | | | |
| 228 | 5-$NO_2$ | 2 | | | |
| 229 | 6-$NO_2$ | 2 | | | |
| 230 | 3-CN | 2 | | | |
| 231 | 4-CN | 2 | | | |
| 232 | 5-CN | 2 | | | |
| 233 | 6-CN | 2 | | | |

Tabelle 2a (Fortsetzung)

| Nr. | $R^4$, $R^5$, $R^6$ | Verknüpfung | Fp ($^oC$) | cis/trans-Verhältnis | H-NMR (ppm) |
|---|---|---|---|---|---|
| 234 | 5,6-[-CH=CH-CH=CH-] | 2 | | | |
| 235 | 5,6-[-CH=CH-CH=CH-] | 3 | | | |
| 236 | 5,6-[-CH=CH-CH=CH-] | 4 | | | |
| 237 | 5,6-[$-(CH_2)_4-$] | 2 | | | |
| 238 | 5,6-[$-(CH_2)_4-$] | 3 | | | |
| 239 | 5,6-[$-(CH_2)_4-$] | 4 | | | |

Tabelle 2b:

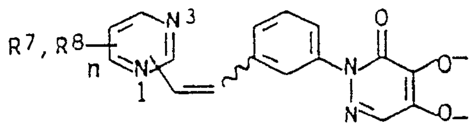

| Nr. | $R^7$, $R^8$ | Ver- knüpfung | Fp (°C) | cis/trans- Verhältnis | H-NMR (ppm) |
|---|---|---|---|---|---|
| 240 | H | 2 | | | |
| 241 | H | 4 | | | |
| 242 | H | 5 | | | |
| 243 | 4-$CH_3$ | 2 | | | |
| 244 | 5-$CH_3$ | 2 | | | |
| 245 | 2-$CH_3$ | 4 | | | |
| 246 | 5-$CH_3$ | 4 | | | |
| 247 | 6-$CH_3$ | 4 | | | |
| 248 | 2-$CH_3$ | 5 | | | |
| 249 | 4-$CH_3$ | 5 | | | |
| 250 | 4-Cl | 2 | | | |
| 251 | 5-Cl | 2 | | | |
| 252 | 2-Cl | 4 | | | |
| 253 | 5-Cl | 4 | | | |
| 254 | 6-Cl | 4 | | | |
| 255 | 2-Cl | 5 | | | |
| 256 | 4-Cl | 5 | | | |
| 257 | 4-$CF_3$ | 2 | | | |
| 258 | 5-$CF_3$ | 2 | | | |
| 259 | 2-$CF_3$ | 4 | | | |
| 260 | 5-$CF_3$ | 4 | | | |
| 261 | 6-$CF_3$ | 4 | | | |
| 262 | 2-$CF_3$ | 5 | | | |
| 263 | 4-$CF_3$ | 5 | | | |
| 264 | 4-$OCH_3$ | 2 | | | |
| 265 | 5-$OCH_3$ | 2 | | | |
| 266 | 2-$OCH_3$ | 4 | | | |
| 267 | 5-$OCH_3$ | 4 | | | |
| 268 | 6-$OCH_3$ | 4 | | | |
| 269 | 2-$OCH_3$ | 5 | | | |
| 270 | 4-$OCH_3$ | 5 | | | |
| 271 | 4-$SCH_3$ | 2 | | | |
| 272 | 5-$SCH_3$ | 2 | | | |
| 273 | 2-$SCH_3$ | 4 | | | |
| 274 | 5-$SCH_3$ | 4 | | | |

Tabelle 2b (Fortsetzung)

| Nr. | $R^7$, $R^8$ | Verknüpfung | Fp ($^oC$) | cis/trans-Verhältnis | H-NMR (ppm) |
|---|---|---|---|---|---|
| 275 | 6-$SCH_3$ | 4 | | | |
| 276 | 2-$SCH_3$ | 5 | | | |
| 277 | 4-$SCH_3$ | 5 | | | |
| 278 | 4-$OCF_3$ | 2 | | | |
| 279 | 5-$OCF_3$ | 2 | | | |
| 280 | 2-$OCF_3$ | 4 | | | |
| 281 | 5-$OCF_3$ | 4 | | | |
| 282 | 6-$OCF_3$ | 4 | | | |
| 283 | 2-$OCF_3$ | 5 | | | |
| 284 | 4-$OCF_3$ | 5 | | | |
| 285 | 4-$SCF_3$ | 2 | | | |
| 286 | 5-$SCF_3$ | 2 | | | |
| 287 | 2-$SCF_3$ | 4 | | | |
| 288 | 5-$SCF_3$ | 4 | | | |
| 289 | 6-$SCF_3$ | 4 | | | |
| 290 | 2-$SCF_3$ | 5 | | | |
| 291 | 4-$SCF_3$ | 5 | | | |
| 292 | 2-$NO_2$ | 5 | | | |
| 293 | 5-$NO_2$ | 4 | | | |
| 294 | 6-$NO_2$ | 4 | | | |
| 295 | 2-CN | 4 | | | |
| 296 | 5-CN | 4 | | | |
| 297 | 6-CN | 4 | | | |
| 298 | 4,5-[-CH=CH-CH=CH-] | 2 | | | |
| 299 | 5,6-[-CH=CH-CH=CH-] | 4 | | | |

Tabelle 2c:

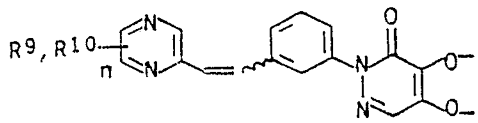

| Nr. | $R^9$, $R^{10}$ | Fp ($^oC$) | cis/trans-Verhältnis | H-NMR (ppm) |
|---|---|---|---|---|
| 300 | H | | | |
| 301 | $3-CH_3$ | | | |
| 302 | $5-CH_3$ | | | |
| 303 | $6-CH_3$ | | | |
| 304 | 3-F | | | |
| 305 | 5-F | | | |
| 306 | 6-F | | | |
| 307 | 3-Cl | | | |
| 308 | 5-Cl | | | |
| 309 | 6-Cl | | | |
| 310 | $3-OCH_3$ | | | |
| 311 | $5-OCH_3$ | | | |
| 312 | $6-OCH_3$ | | | |
| 313 | $3-CF_3$ | | | |
| 314 | $5-CF_3$ | | | |
| 315 | $6-CF_3$ | | | |
| 316 | $3-OCF_3$ | | | |
| 317 | $5-OCF_3$ | | | |
| 318 | $6-OCF_3$ | | | |
| 319 | $3-SCH_3$ | | | |
| 320 | $5-SCH_3$ | | | |
| 321 | $6-SCH_3$ | | | |
| 322 | $3-SCF_3$ | | | |
| 323 | $5-SCF_3$ | | | |
| 324 | $6-SCF_3$ | | | |
| 325 | $3-NO_2$ | | | |
| 326 | $5-NO_2$ | | | |
| 327 | $6-NO_2$ | | | |
| 328 | 3-CN | | | |
| 329 | 5-CN | | | |
| 330 | 6-CN | | | |
| 331 | 5,6-[-CH=CH-CH=CH-] | | | |
| 332 | $3,5-Cl_2$ | | | |
| 333 | $3,6-Cl_2$ | | | |
| 334 | $5,6-Cl_2$ | | | |
| 335 | $3,5-(CH_3)_2$ | | | |
| 336 | $3,6-(CH_3)_2$ | | | |
| 337 | $5,6-(CH_3)_2$ | | | |

Tabelle 2d:

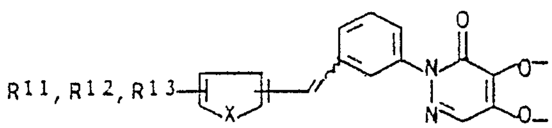

| Nr. | R11,R12,R13 | X | Verknüpfung | Fp(°C) | cis/trans-Verhältnis | H-NMR (ppm) |
|---|---|---|---|---|---|---|
| 338 | H | O | 2 | Oel | cis/trans = 1/1 | cis: 7,82 (s; 1H) trans: 7,84 (s; 1 H) |
| 339 | H | O | 3 | | | |
| 340 | 3-$CH_3$ | O | 2 | | | |
| 341 | 4-$CH_3$ | O | 2 | | | |
| 342 | 5-$CH_3$ | O | 2 | | | |
| 343 | 2-$CH_3$ | O | 3 | | | |
| 344 | 4-$CH_3$ | O | 3 | | | |
| 345 | 5-$CH_3$ | O | 3 | | | |
| 346 | 3-Cl | O | 2 | | | |
| 247 | 4-Cl | O | 2 | | | |
| 348 | 5-Cl | O | 2 | | | |
| 349 | 3-Br | O | 2 | | | |
| 350 | 4-Br | O | 2 | | | |
| 351 | 5-Br | O | 2 | | | |
| 352 | 2-Cl | O | 3 | | | |
| 353 | 4-Cl | O | 3 | | | |
| 354 | 5-Cl | O | 3 | | | |
| 355 | 2-Br | O | 3 | | | |
| 356 | 4-Br | O | 3 | | | |
| 357 | 5-Br | O | 3 | | | |
| 358 | 3,4-$Cl_2$ | O | 2 | | | |
| 359 | 3,5-$Cl_2$ | O | 2 | | | |
| 360 | 4,5-$Cl_2$ | O | 2 | | | |
| 361 | 2,4-$Cl_2$ | O | 3 | | | |
| 362 | 2,5-$Cl_2$ | O | 3 | | | |
| 363 | 4,5-$Cl_2$ | O | 3 | | | |
| 364 | 3,4-$Br_2$ | O | 2 | | | |
| 365 | 3,5-$Br_2$ | O | 2 | | | |
| 366 | 4,5-$Br_2$ | O | 2 | | | |
| 367 | 2,4-$Br_2$ | O | 3 | | | |
| 368 | 2,5-$Br_2$ | O | 3 | | | |
| 369 | 4,5-$Br_2$ | O | 3 | | | |
| 370 | 3,4,5-$Cl_3$ | O | 2 | | | |

Tabelle 2d (Fortsetzung):

| Nr. | $R^{11}, R^{12}, R^{13}$ | X | Verknüpfung | Fp(°C) | cis/trans-Verhältnis | H-NMR (ppm) |
|---|---|---|---|---|---|---|
| 371 | 2,4,5-$Cl_3$ | O | 3 | | | |
| 372 | 3,4,5-$Br_3$ | O | 2 | | | |
| 373 | 2,4,5-$Br_3$ | O | 3 | | | |
| 374 | 3-$OCH_3$ | O | 2 | | | |
| 375 | 4-$OCH_3$ | O | 2 | | | |
| 376 | 5-$OCH_3$ | O | 2 | | | |
| 377 | 2-$OCH_3$ | O | 3 | | | |
| 378 | 4-$OCH_3$ | O | 3 | | | |
| 379 | 5-$OCH_3$ | O | 3 | | | |
| 380 | 2,5-$(OCH_3)_2$ | O | 3 | | | |
| 381 | 3-$OCF_3$ | O | 2 | | | |
| 382 | 4-$OCF_3$ | O | 2 | | | |
| 383 | 5-$OCF_3$ | O | 2 | | | |
| 384 | 2-$OCF_3$ | O | 3 | | | |
| 385 | 4-$OCF_3$ | O | 3 | | | |
| 386 | 5-$OCF_3$ | O | 3 | | | |
| 387 | 3-$SCH_3$ | O | 2 | | | |
| 388 | 4-$SCH_3$ | O | 2 | | | |
| 389 | 5-$SCH_3$ | O | 2 | | | |
| 390 | 2-$SCH_3$ | O | 3 | | | |
| 391 | 4-$SCH_3$ | O | 3 | | | |
| 392 | 5-$SCH_3$ | O | 3 | | | |
| 393 | 3-$SCF_3$ | O | 2 | | | |
| 394 | 4-$SCF_3$ | O | 2 | | | |
| 395 | 5-$SCF_3$ | O | 2 | | | |
| 396 | 2-$SCF_3$ | O | 3 | | | |
| 397 | 4-$SCF_3$ | O | 3 | | | |
| 398 | 5-$SCF_3$ | O | 3 | | | |
| 399 | 3-$NO_2$ | O | 2 | | | |
| 400 | 4-$NO_2$ | O | 2 | | | |
| 401 | 5-$NO_2$ | O | 2 | | | |
| 402 | 2-$NO_2$ | O | 3 | | | |
| 403 | 4-$NO_2$ | O | 3 | | | |
| 404 | 5-$NO_2$ | O | 3 | | | |
| 405 | 3-CN | O | 2 | | | |
| 406 | 4-CN | O | 2 | | | |
| 407 | 5-CN | O | 2 | | | |
| 408 | 2-CN | O | 3 | | | |
| 409 | 4-CN | O | 3 | | | |
| 410 | 5-CN | O | 3 | | | |

Tabelle 2d (Fortsetzung):

| Nr. | R11, R12, R13 | X | Verknüpfung | Fp(°C) | cis/trans-Verhältnis | H-NMR (ppm) |
|---|---|---|---|---|---|---|
| 411 | 3,4-[-CH=CH-CH=CH-] | O | 2 | | | |
| 412 | 4,5-[-CH=CH-CH=CH-] | O | 2 | | | |
| 413 | 4,5-[-CH=CH-CH=CH-] | O | 3 | | | |
| 414 | H | S | 2 | Oel | cis | 7,80 (s; 1 H) |
| 415 | H | S | 2 | 101-102 | trans | |
| 416 | H | S | 3 | | | |
| 417 | 3-$CH_3$ | S | 2 | | | |
| 418 | 4-$CH_3$ | S | 2 | | | |
| 419 | 5-$CH_3$ | S | 2 | | | |
| 420 | 2-$CH_3$ | S | 3 | | | |
| 421 | 4-$CH_3$ | S | 3 | | | |
| 422 | 5-$CH_3$ | S | 3 | | | |
| 423 | 3-Cl | S | 2 | | | |
| 424 | 4-Cl | S | 2 | | | |
| 425 | 5-Cl | S | 2 | | | |
| 426 | 3-Br | S | 2 | | | |
| 427 | 4-Br | S | 2 | | | |
| 428 | 5-Br | S | 2 | | | |
| 429 | 2-Cl | S | 3 | | | |
| 430 | 4-Cl | S | 3 | | | |
| 431 | 5-Cl | S | 3 | | | |
| 432 | 2-Br | S | 3 | | | |
| 433 | 4-Br | S | 3 | | | |
| 434 | 5-Br | S | 3 | | | |
| 435 | 3,4-$Cl_2$ | S | 2 | | | |
| 436 | 3,5-$Cl_2$ | S | 2 | | | |
| 437 | 4,5-$Cl_2$ | S | 2 | | | |
| 438 | 2,4-$Cl_2$ | S | 3 | | | |
| 439 | 2,5-$Cl_2$ | S | 3 | | | |
| 440 | 4,5-$Cl_2$ | S | 3 | | | |
| 441 | 3,4-$Br_2$ | S | 2 | | | |
| 442 | 3,5-$Br_2$ | S | 2 | | | |
| 443 | 4,5-$Br_2$ | S | 2 | | | |
| 444 | 2,4-$Br_2$ | S | 3 | | | |
| 445 | 2,5-$Br_2$ | S | 3 | | | |
| 446 | 4,5-$Br_2$ | S | 3 | | | |
| 447 | 3,4,5-$Cl_3$ | S | 2 | | | |
| 448 | 2,4,5-$Cl_3$ | S | 3 | | | |
| 449 | 3,4,5-$Br_3$ | S | 2 | | | |
| 450 | 2,4,5-$Br_3$ | S | 3 | | | |
| 451 | 3-$OCH_3$ | S | 2 | | | |

Tabelle 2d (Fortsetzung):

| Nr. | R11, R12, R13 | X | Ver-knüpfung | Fp(°C) | cis/trans-Verhältnis | H-NMR (ppm) |
|---|---|---|---|---|---|---|
| 452 | 4-$OCH_3$ | S | 2 | | | |
| 453 | 5-$OCH_3$ | S | 2 | | | |
| 454 | 2-$OCH_3$ | S | 3 | | | |
| 455 | 4-$OCH_3$ | S | 3 | | | |
| 456 | 5-$OCH_3$ | S | 3 | | | |
| 457 | 2,5-$(OCH_3)_2$ | S | 3 | | | |
| 458 | 3-$OCF_3$ | S | 2 | | | |
| 459 | 4-$OCF_3$ | S | 2 | | | |
| 460 | 5-$OCF_3$ | S | 2 | | | |
| 461 | 2-$OCF_3$ | S | 3 | | | |
| 462 | 4-$OCF_3$ | S | 3 | | | |
| 463 | 5-$OCF_3$ | S | 3 | | | |
| 464 | 3-$SCH_3$ | S | 2 | | | |
| 465 | 4-$SCH_3$ | S | 2 | | | |
| 466 | 5-$SCH_3$ | S | 2 | | | |
| 467 | 2-$SCH_3$ | S | 3 | | | |
| 468 | 4-$SCH_3$ | S | 3 | | | |
| 469 | 5-$SCH_3$ | S | 3 | | | |
| 470 | 3-$SCF_3$ | S | 2 | | | |
| 471 | 4-$SCF_3$ | S | 2 | | | |
| 472 | 5-$SCF_3$ | S | 2 | | | |
| 473 | 2-$SCF_3$ | S | 3 | | | |
| 474 | 4-$SCF_3$ | S | 3 | | | |
| 475 | 5-$SCF_3$ | S | 3 | | | |
| 476 | 3-$NO_2$ | S | 2 | | | |
| 477 | 4-$NO_2$ | S | 2 | | | |
| 478 | 5-$NO_2$ | S | 2 | | | |
| 479 | 2-$NO_2$ | S | 3 | | | |
| 480 | 4-$NO_2$ | S | 3 | | | |
| 481 | 5-$NO_2$ | S | 3 | | | |
| 482 | 3-CN | S | 2 | | | |
| 483 | 4-CN | S | 2 | | | |
| 484 | 5-CN | S | 2 | | | |
| 485 | 2-CN | S | 3 | | | |
| 486 | 4-CN | S | 3 | | | |
| 487 | 5-CN | S | 3 | | | |
| 488 | 3,4-[-CH=CH-CH=CH-] | S | 2 | | | |
| 489 | 4,5-[-CH=CH-CH=CH-] | S | 2 | | | |
| 490 | 4,5-[-CH=CH-CH=CH-] | S | 3 | | | |

Tabelle 2d (Fortsetzung):

| Nr. | $R^{11}$, $R^{12}$, $R^{13}$ | X | Verknüpfung | Fp(°C) | cis/trans-Verhältnis | H-NMR (ppm) |
|---|---|---|---|---|---|---|
| 491 | H | $N-CH_3$ | 2 | | | |
| 492 | H | $N-C_2H_5$ | 2 | | | |
| 493 | H | $N-nC_3H_7$ | 2 | | | |
| 494 | H | $N-iC_3H_7$ | 2 | | | |
| 495 | H | $N-CH_2C_6H_5$ | 2 | | | |
| 496 | H | $N-C_6H_5$ | 2 | | | |
| 497 | H | $N-4-Cl-C_6H_5$ | 2 | | | |
| 498 | H | $NCH_3$ | 3 | | | |
| 499 | H | $NC_2H_5$ | 3 | | | |
| 500 | H | $NnC_3H_7$ | 3 | | | |
| 501 | H | $N-iC_3H_7$ | 3 | | | |
| 502 | H | $N-CH_2C_6H_5$ | 3 | | | |
| 503 | H | $N-C_6H_5$ | 3 | | | |
| 504 | H | $N-4-ClC_6H_5$ | 3 | | | |
| 505 | 4,5-[-CH=CH-CH=CH-] | $N-CH_3$ | 2 | | | |
| 506 | 4,5-[-CH=CH-CH=CH-] | $N-CH_2C_6H_5$ | 2 | | | |
| 507 | 4,5-[-CH=CH-CH=CH-] | $N-CH_3$ | 3 | | | |
| 508 | 4,5-[-CH=CH-CH=CH-] | $N-CH_2C_6H_5$ | 3 | | | |

Tabelle 2e:

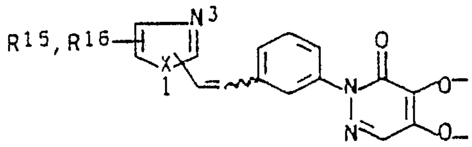

| Nr. | R15, R16 | X | Ver-knüpfung | Fp($^{o}C$) | cis/trans-Verhältnis | H-NMR (ppm) |
|---|---|---|---|---|---|---|
| 509 | H | O | 2 | | | |
| 510 | 4-$CH_3$ | O | 2 | | | |
| 511 | 5-$CH_3$ | O | 2 | | | |
| 512 | 2-$CH_3$ | O | 4 | | | |
| 513 | 2-$CH_3$ | O | 5 | | | |
| 514 | 4-$CF_3$ | O | 2 | | | |
| 515 | 5-$CF_3$ | O | 2 | | | |
| 516 | 2-$CF_3$ | O | 4 | | | |
| 517 | 2-$CF_3$ | O | 5 | | | |
| 518 | 2-$OCH_3$ | O | 5 | | | |
| 519 | 2-$SCF_3$ | O | 5 | | | |
| 520 | 2-Cl | O | 5 | | | |
| 521 | 2-$NO_2$ | O | 5 | | | |
| 522 | 2-CN | O | 5 | | | |
| 523 | 4,5-[-CH=CH-CH=CH-] | O | 2 | | | |
| 524 | 4,5-[-$(CH_3)_2$-] | O | 2 | | | |
| 525 | H | S | 2 | | | |
| 526 | 4-$CH_3$ | S | 2 | | | |
| 527 | 5-$CH_3$ | S | 2 | | | |
| 528 | 2-$CH_3$ | S | 4 | | | |
| 529 | 2-$CH_3$ | S | 5 | | | |
| 530 | 4-$CF_3$ | S | 2 | | | |
| 531 | 5-$CF_3$ | S | 2 | | | |
| 532 | 2-$CF_3$ | S | 4 | | | |
| 533 | 2-$CF_3$ | S | 5 | | | |
| 534 | 2-$OCF_3$ | S | 5 | | | |
| 535 | 2-$SCF_3$ | S | 5 | | | |
| 536 | 2-Cl | S | 5 | | | |
| 537 | 2-$NO_2$ | S | 5 | | | |
| 538 | 2-CN | S | 5 | | | |
| 539 | 4,5-$(CH_3)_2$ | S | 2 | | | |

Tabelle 2e (Fortsetzung):

| Nr. | $R^{15}$,$R^{16}$ | X | Verknüpfung | Fp(°C) | cis/trans-Verhältnis | H-NMR (ppm) |
|---|---|---|---|---|---|---|
| 540 | 4,5-[-CH=CH-CH=CH-] | S | 2 | | | |
| 541 | H | $NCH_3$ | 2 | | | |
| 542 | 4-$CH_3$ | $NCH_3$ | 2 | | | |
| 543 | 5-$CH_3$ | $NCH_3$ | 2 | | | |
| 544 | 2-$CH_3$ | $NCH_3$ | 4 | | | |
| 545 | 2-$CH_3$ | $NCH_3$ | 5 | | | |
| 546 | 4-$CF_3$ | $NCH_3$ | 2 | | | |
| 547 | 5-$CF_3$ | $NCH_3$ | 2 | | | |
| 548 | 2-$CF_3$ | $NCH_3$ | 4 | | | |
| 549 | 2-$CF_3$ | $NCH_3$ | 5 | | | |
| 550 | 2-$OCF_3$ | $NCH_3$ | 4 | | | |
| 551 | 2-$SCF_3$ | $NCH_3$ | 4 | | | |
| 552 | 2-$OCH_3$ | $NCH_3$ | 4 | | | |
| 553 | 2-$SCH_3$ | $NCH_3$ | 4 | | | |
| 554 | 2-Cl | $NCH_3$ | 4 | | | |
| 555 | 2-$NO_2$ | $NCH_3$ | 4 | | | |
| 556 | 2-CN | $NCH_3$ | 4 | | | |
| 557 | 4,5-[-$(CH_3)_2$-] | $NCH_3$ | 2 | | | |
| 558 | 4,5-[-CH=CH-CH=CH-] | $NCH_3$ | 2 | | | |

Tabelle 2f:

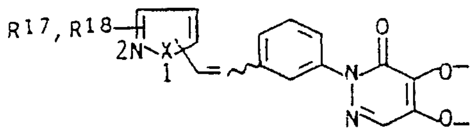

| Nr. | R17, R18 | X | Verknüpfung | Fp(°C) | cis/trans-Verhältnis | H-NMR (ppm) |
|---|---|---|---|---|---|---|
| 559 | H | O | 5 | | | |
| 560 | 3-$CH_3$ | O | 5 | | | |
| 561 | 3-$C_2H_5$ | O | 5 | 50-54 | cis | |
| 562 | 3-$nC_3H_7$ | O | 5 | | | |
| 563 | 3-$iC_3H_7$ | O | 5 | Oel | cis/trans = 2/1 | cis: 7,81 (s; 1H) trans: 7,86 (s; 1H) |
| 564 | 3-$nC_4H_9$ | O | 5 | | | |
| 565 | 3-$iC_4H_9$ | O | 5 | Oel | cis | 7,82 (s; 1 H) |
| 566 | 3-sec$C_4H_9$ | O | 5 | | | |
| 567 | 3-$tC_4H_9$ | O | 5 | | | |
| 568 | 3-n-$C_5H_{11}$ | O | 5 | | | |
| 569 | 3-n-$C_6H_{13}$ | O | 5 | | | |
| 570 | 3-$C_6H_5$ | O | 5 | | | |
| 571 | 3-4'Cl-$C_6H_4$ | O | 5 | | | |
| 572 | 3-4'$CH_3$-$C_6H_4$ | O | 5 | | | |
| 573 | 3-4'$CF_3$-$C_6H_4$ | O | 5 | | | |
| 574 | 3-$CH_3OCH_2$ | O | 5 | | | |
| 575 | 3-2'Furyl | O | 5 | | | |
| 576 | 3-3'Furyl | O | 5 | | | |
| 577 | 3-2'Thienyl | O | 5 | | | |
| 578 | 3-3'Thienyl | O | 5 | | | |
| 579 | 3-(2'-Tetrahydropyranyl) | O | 5 | | | |
| 580 | 3-(3'-Tetrahydropyranyl) | O | 5 | | | |
| 581 | 3-(4'-Tetrahydropyranyl) | O | 5 | | | |
| 582 | 3-(2'-Tetrahydrothiopyranyl) | O | 5 | | | |
| 583 | 3-(3'-Tetrahydrothiopyranyl) | O | 5 | | | |

Tabelle 2f (Fortsetzung):

| Nr. | $R^{17}, R^{18}$ | X | Ver-knüpfung | Fp($^oC$) | cis/trans-Verhältnis | H-NMR (ppm) |
|---|---|---|---|---|---|---|
| 584 | 3-(4'-Tetrahydrothiopyranyl) | O | 5 | | | |
| 585 | 3-cyclo-$C_3H_5$ | O | 5 | | | |
| 586 | 3-cyclo-$C_4H_7$ | O | 5 | | | |
| 587 | 3-cyclo-$C_5H_9$ | O | 5 | | | |
| 588 | 3-cyclo-$C_6H_{11}$ | O | 5 | | | |
| 589 | 3-cyclo-$C_7H_{13}$ | O | 5 | | | |
| 590 | 3,5($CH_3$)$_2$ | O | 4 | | | |
| 591 | 3-$CH_3$ | S | 5 | | | |
| 592 | 3-$C_2H_5$ | S | 5 | | | |
| 593 | 3-n$C_3H_7$ | S | 5 | | | |
| 594 | 3-i$C_3H_7$ | S | 5 | | | |
| 595 | H | $NCH_3$ | 4 | Oel | cis/trans = 1/2 | cis: 7,80 (s; 1H); trans: 7,83 (s; 1H) |
| 596 | H | N-$C_4H_9$ | 4 | | | |
| 597 | 3-$CH_3$ | $NCH_3$ | 4 | | | |
| 598 | 3-$CH_3$ | $NCH_3$ | 5 | | | |
| 599 | 5-$CH_3$ | $NCH_3$ | 4 | | | |

Tabelle 2g:

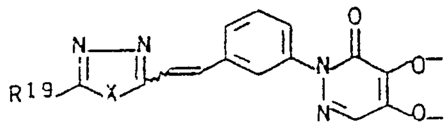

| Nr. | R19 | X | Fp(°C) | cis/trans-Verhältnis | H-NMR (ppm) |
|---|---|---|---|---|---|
| 600 | H | O | | | |
| 601 | $CH_3$ | O | | | |
| 602 | $C_2H_5$ | O | | | |
| 603 | $nC_3H_7$ | O | | | |
| 604 | $n-C_4H_9$ | O | | | |
| 605 | $t-C_4H_9$ | O | | | |
| 606 | $CF_3$ | O | | | |
| 607 | $OCH_3$ | O | | | |
| 608 | $OCF_3$ | O | | | |
| 609 | $SCH_3$ | O | | | |
| 610 | $SCF_3$ | O | | | |
| 611 | H | S | | | |
| 612 | $CH_3$ | S | | | |
| 613 | $C_2H_5$ | S | | | |
| 614 | $n-C_3H_7$ | S | | | |
| 615 | $n-C_4H_9$ | S | | | |
| 616 | $t-C_4H_9$ | S | | | |
| 617 | $CF_3$ | S | | | |
| 618 | $OCH_3$ | S | | | |
| 619 | $OCF_3$ | S | | | |
| 620 | $SCH_3$ | S | | | |
| 621 | $SCF_3$ | S | | | |

Tabelle 2h:

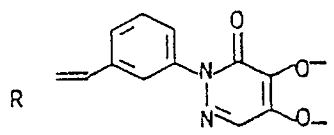

| Nr. | R | Fp(°C) | cis/trans-Verhältnis | H-NMR (ppm) |
|---|---|---|---|---|
| 622 | Me | | | |
| 623 | Et | Oel | cis/trans = 1/1 | cis: 7,81 (s; 1 H), trans: 7,82 (s; 1 H) |
| 624 | $n-C_3H_7$ | | | |
| 625 | $i-C_3H_7$ | | | |
| 626 | $n-C_4H_9$ | Oel | cis/trans = 1/1 | cis: 7,80 (s; 1H); trans: 7,81 (s; 1H) |
| 627 | $i-C_4H_9$ | | | |
| 628 | $sec-C_4H_9$ | | | |
| 629 | $t-C_4H_9$ | | | |
| 630 | $n-C_5H_{11}$ | | | |
| 631 | $n-C_6H_{13}$ | | | |
| 632 | $n-C_7H_{15}$ | | | |
| 633 | $n-C_8H_{17}$ | | | |
| 634 | cyclo-$C_3H_5$ | Oel | cis/trans = 1/1 | cis: 7,81 (s; 1H); trans: 7,82 (s; 1H) |
| 635 | cyclo-$C_4H_7$ | | | |
| 636 | cyclo-$C_5H_9$ | | | |
| 637 | cyclo-$C_6H_{11}$ | | | |
| 638 | cyclo-$C_7H_{13}$ | | | |
| 639 | cyclo-$C_8H_{15}$ | | | |
| 640 | 2-Norbornyl | | | |
| 641 | 2-Tetrahydrofuryl | | | |
| 642 | 3-Tetrahydrofuryl | | | |
| 643 | 2-Tetrahydropyranyl | | | |
| 644 | 3-Tetrahydropyranyl | | | |
| 645 | 4-Tetrahydropyranyl | | | |
| 646 | 2-Tetrahydrothienyl | | | |
| 647 | 3-Tetrahydrothienyl | | | |
| 648 | 2-Tetrahydrothiopyranyl | | | |
| 649 | 3-Tetrahydrothiopyranyl | | | |

Tabelle 2h (Fortsetzung):

| Nr. | R | Fp(°C) | cis/trans-Verhältnis | H-NMR (ppm) |
|---|---|---|---|---|
| 650 | 4-Tetrahydrothiopyranyl | | | |
| 651 | 2-Dioxanyl-(1,4)- | | | |
| 652 | Benzyl | Oel | cis/trans = 1/9 | cis: 7,79 (s;1H); trans: 7,81 (s;1H) |
| 653 | 2-F-Benzyl | | | |
| 654 | 3-F-Benzyl | | | |
| 655 | 4-F-Benzyl | | | |
| 656 | 2-Cl-Benzyl | | | |
| 657 | 3-Cl-Benzyl | | | |
| 658 | 4-Cl-Benzyl | | | |
| 659 | 2-$CH_3$-Benzyl | | | |
| 660 | 3-$CH_3$-Benzyl | | | |
| 661 | 4-$CH_3$-Benzyl | | | |
| 662 | 2-$CF_3$-Benzyl | | | |
| 663 | 3-$CF_3$-Benzyl | | | |
| 664 | 4-$CF_3$-Benzyl | | | |
| 665 | 2-$OCH_3$-Benzyl | | | |
| 666 | 3-$OCH_3$-Benzyl | | | |
| 667 | 4-$OCH_3$-Benzyl | | | |
| 668 | 2-$OCF_3$-Benzyl | | | |
| 669 | 3-$OCF_3$-Benzyl | | | |
| 670 | 4-$OCF_3$-Benzyl | | | |
| 671 | 2-$SCH_3$-Benzyl | | | |
| 672 | 3-$SCH_3$-Benzyl | | | |
| 673 | 4-$SCH_3$-Benzyl | | | |
| 674 | 2-$SCF_3$-Benzyl | | | |
| 675 | 3-$SCF_3$-Benzyl | | | |
| 676 | 4-$SCF_3$-Benzyl | | | |
| 677 | 2-CN-Benzyl | | | |
| 678 | 3-CN-Benzyl | | | |
| 679 | 4-CN-Benzyl | | | |
| 680 | Styryl | 134-140 | trans-trans | |
| 681 | Styryl | 83-86 | trans-cis | |
| 682 | 2-F-Styryl | | | |
| 683 | 3-F-Styryl | | | |
| 684 | 4-F-Styryl | | | |
| 685 | 2-Cl-Styryl | | | |
| 686 | 3-Cl-Styryl | | | |

Tabelle 2h (Fortsetzung):

| Nr. | R | Fp(°C) | cis/trans-Verhältnis | H-NMR (ppm) |
|---|---|---|---|---|
| 687 | 4-Cl-Styryl | | | |
| 688 | 2-$CH_3$-Styryl | | | |
| 689 | 3-$CH_3$-Styryl | | | |
| 690 | 4-$CH_3$-Styryl | | | |
| 691 | 2-$CF_3$-Styryl | | | |
| 692 | 3-$CF_3$-Styryl | | | |
| 693 | 4-$CF_3$-Styryl | | | |
| 694 | 2-$OCH_3$-Styryl | | | |
| 695 | 3-$OCH_3$-Styryl | | | |
| 696 | 4-$OCH_3$-Styryl | | | |
| 697 | 2-$OCF_3$-Styryl | | | |
| 698 | 3-$OCF_3$-Styryl | | | |
| 699 | 4-$OCF_3$-Styryl | | | |
| 700 | 2-$SCH_3$-Styryl | | | |
| 701 | 3-$SCH_3$-Styryl | | | |
| 702 | 4-$SCH_3$-Styryl | | | |
| 703 | 2-$SCF_3$-Styryl | | | |
| 704 | 3-$SCF_3$-Styryl | | | |
| 705 | 4-$SCF_3$-Styryl | | | |
| 706 | 2-CN-Styryl | | | |
| 707 | 3-CN-Styryl | | | |
| 708 | 4-CN-Styryl | | | |
| 709 | 2-$NO_2$-Styryl | | | |
| 710 | 3-$NO_2$-Styryl | | | |
| 711 | 4-$NO_2$-Styryl | | | |

Anwendungsbeispiele:

Die herbizide Wirkung auf das Wachstum der Testpflanzen wird durch folgende Gewächshausversuche gezeigt:
Zur Anzucht der Testpflanzen dienen Plastikblumentöpfe mit 300 $cm^3$ Inhalt und lehmigem Sand mit etwa 3,0 % Humus als Substrat. Die Samen der Testpflanzen werden getrennt eingsät.

Zum Zweck der Nachauflaufanwendung werden entweder direkt gesäte oder in den gleichen Gefäßen aufgewachsene Pflanzen ausgewählt oder sie werden erst als Keimpflanzen getrennt angezogen und einige Tage vor der Behandlung in die Versuchsgefäße verpflanzt.

Je nach Wuchsform werden die Testpflanzen bei einer Wuchshöhe von 3-15 cm dann mit den in Wassr als Verteilungsmittel suspendierten oder emulgierten Wirkstoffen, die durch fein verteilende Düsen gespritzt werden, behandelt. Die Aufwandmenge für die Nachauflaufbehandlung beträgt 0,06 kg/ha a.S.

Die Versuchsgefäße werden im Gewächshaus aufgestellt, wobei für wärmeliebende Arten wärmere Bereiche (20 bis 35 °C) und für solche gemäßigter Klimate 10 bis 20 °C bevorzugt werden. Die Versuchsperiode erstreckt sich über 2 bis 4 Wochen. Während dieser Zeit werden die Pflanzen gepflegt und ihre

Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzen sich aus folgenden Arten zusammen:

| Abkürzung | lateinischer Name | deutscher Name | englischer Name |
|---|---|---|---|
| AMARE | Amaranthus retroflexus | Zurückgekrümmter Fuchsschwanz | redroot pigweed |
| CASTO | Cassia tora | - | sicklepod |
| CHEAL | Chenopodium album | Weißer Gänsefuß | lampsquarters (goosegoot) |
| GOSHI | Gossypium hirsutum | Baumwolle | cotton |
| HELAN | Helianthus annus | Sonnenblume | sunflowers |

Als Vergleichsmittel werden Wirkstoffe verwendet, die in der DE-A-3 617 997 beschrieben sind.

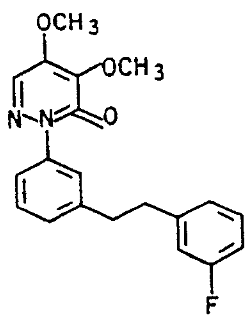

A (Wirkstoff-Nr. 30 aus DE-A 3 617 997)

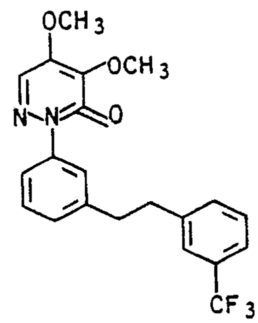

B (Wirkstoff-Nr. 27 aus DE-A 3 617 997)

Mit 0,06 kg/ha a.S. im Nachauflaufverfahren eingesetzt, lassen sich mit Beispiel 43 bzw. Beispiel breitblättrige unerwünschte Pflanzen sehr gut bekämpfen, und zwar im Vergleich zu den bekannten Herbiziden A und B mit deutlich besserer Verträglichkeit für Baumwolle une Sonnenblume.

## Ansprüche

1. 2-Phenylpyridazin-3-on-Verbindungen der Formeln

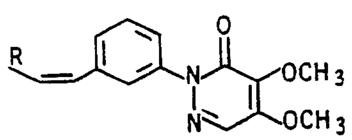

Ia

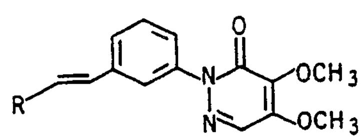

Ib

in der R die folgende Bedeutung hat:
eine $C_1$-$C_8$-Alkylgruppe;
eine Benzylgruppe, wobei der aromatische Ring ein bis drei der folgenden Substituenten tragen kann: Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio und/oder Cyano;
eine $C_3$-$C_8$-Cycloalkylgruppe, welche ein bis drei der folgenden Substituenten tragen kann: Halogen, $C_1$-$C_4$-Alkyl, $C_1$-$C_4$-Halogenalkyl, $C_1$-$C_4$-Alkoxy und/oder $C_1$-$C_4$-Halogenalkoxy;
ein 4- bis 6-gliedriger heteroaliphatischer Ring, enthaltend ein Sauerstoff- oder Schwefelatom oder eine N-$CH_3$-Gruppe als Ringglied;
ein Styrylrest, wobei der aromatische Ring ein bis drei der folgenden Substituenten tragen kann: Nitro,

Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio und/oder Cyano;

ein 5- oder 6-gliedriger aromatischer Ring, welcher ein bix zwei Stickstoff-, Sauerstoff- und/oder Schwefelatome als Ringglieder enthalten kann, wobei dieser Ring zusätzlich benzokondensiert sein kann und ein bis vier der folgenden Substituenten tragen kann:

$C_1$-$C_6$-Alkyl, $C_1$-$C_6$-Halogenalkyl, $C_1$-$C_6$-Alkoxy, $C_1$-$C_6$-Halogenalkoxy, $C_1$-$C_6$-Alkylthio, $C_1$-$C_6$-Halogenalkylthio, Halogen, Nitro, Cyano, Amino, Di-$C_1$-$C_4$-alkylamino, $C_2$-$C_6$-Acylamino, $C_1$-$C_4$-Alkoxycarbonyl, Phenoxy, Phenylthio und/oder eine ortho-ständig an den aromatischen Ring gebundene Brücke

$-X_n-(CH_2)_m-X_n-$,

wobei

X ein Sauerstoff- oder Schwefelatom,

n 1 und gleichzeitig m 1 oder 2 oder

n 0 und gleichzeitig m 3, 4 oder 5

bedeutet.

2. Verbindung nach Anspruch 1, in der R substituieertes Phenyl der Struktur

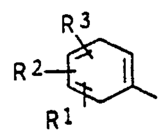

bedeutet, wobei $R^1$, $R^2$ und $R^3$ unabhängig voneinander sein können:

Wasserstoff, $C_1$-$C_6$-Alkyl, $C_{1-6}$-Halogenalkyl, $C_1$-$C_7$-Alkoxy, $C_1$-$C_6$-Halogenalkoxy, $C_1$-$C_6$-Alkylthio, $C_1$-$C_6$-Halogenalkylthio, Phenyl, substituiertes oder unsubstiuiertes Aryloxy, substituiertes oder unsubstituiertes Arylthio, Halogen, Nitro, Amino, Dialkylamino, Acylamino, Diacylamino, Cyano oder Carbonester und ferner $R^1$ und $R^2$ gemeinsam $-X_n-(CH_2)_m-X_n-$, wobei X = Sauerstoff oder Schwefel, n = 0 oder 1 und m = 1,2 wenn n = 1 oder m = 3,4,5 wenn n = 0 ist, sein können, oder gemeinsam mit dem Phenylkern Naphthyl bedeuten.

3. Verfahren zur Herstellung der Verbindungen der Formel I, dadurch gekennzeichnet, daß man ein entsprechendes 2-(3-Methylphenyl)-pyridazinon der Formel V

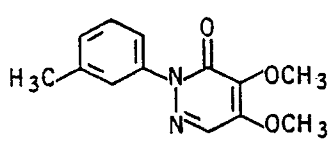

V

in an sich bekannter Weise zu einer Halogenverbindung der Formel V umsetzt,

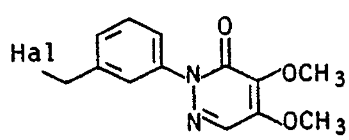

VI

die Halogenverbindung der Formel VI

a) in an sich bekannter Weise in ein entsprechendes Phosphoniumsalz der Formel II

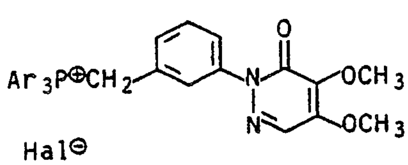

II

oder

b) in ein entsprechendes Phosphonat der Formel IV

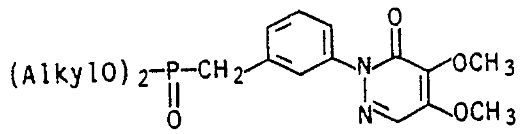

IV

überführt und das Phosphoniumsalz der Formel II oder das Phosphonat der Formel IV mit einem entsprechenden Aldehyd der Formel III

R-CHO III

in Gegenwart einer Base umsetzt.

4. Verwendung der 2-Phenylpyridazin-3-on-derivate der Formeln Ia und Ib gemäß Anspruch 1 als Herbizide.

5. Herbizide Mittel, enthaltend ein 2-Phenylpyridazin-3-on-derivat der Formeln Ia und/oder Ib gemäß Anspruch 1 und inerte Zusatzstoffe.

6. Herbizide Mittel gemäß Anspruch 5, enthaltend weitere wirksame Bestandteile.

7. Verfahren zur Bekämpfung unerwünschten Pflanzenwuchses, dadurch gekennzeichnet, daß man die unerwünschten Pflanzen und/oder ihren Lebensraum mit einer herbizid wirksamen Menge eines 2-Phenylpyridazin 3-on-derivates I und/oder Ib gemäß Anspruch 1 behandelt.

Europäisches Patentamt

# EUROPÄISCHER RECHERCHENBERICHT

Nummer der Anmeldung

EP 89 10 3748

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 247 551 (BASF AG)<br>* Ansprüche 1,6-9 *; & DE - A - 3617997 (Cat. D,A)<br>--- | 1,2,4-7 | C 07 D 237/14<br>A 01 N 43/58 |
| A | EP-A-0 128 530 (BASF AG)<br>* Ansprüche 1,7-10 *<br>--- | 1,2,4,5,7 | |
| A | DE-A-2 037 265 (BASF AG)<br>* Seite 17, letzte Zeile; Seite 28, letzte Zeile; Seite 33, Beispiel 10, Komponente VIII*; & FR - A - 2099642 (Cat. D,A)<br>--- | 1,2,4,6 | |
| A | EP-A-0 031 796 (CIBA-GEIGY AG)<br>* Seiten 31,32; Beispiel 5 *<br>----- | 3 | |

RECHERCHIERTE SACHGEBIETE (Int. Cl.4)

C 07 D 237/00

Der vorliegende Recherchenbericht wurde für alle Patentansprüche erstellt

| Recherchenort | Abschlußdatum der Recherche | Prüfer |
|---|---|---|
| BERLIN | 02-06-1989 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)